# EUROPÄISCHE PATENTSCHRIFT

(11) **EP 0 811 598 B1**
(45) Veröffentlichungstag und Bekanntmachung des Hinweises auf die Patenterteilung: **05.01.2000**
(21) Anmeldenummer: 97108861.2
(22) Anmeldetag: 03.06.1997
(51) Int. Cl.: C07C 69/007, C07C 67/00, A23L 1/226

(54) **5-Z-Octenylester, Verfahren zu ihrer Herstellung und ihre Verwendung**
5-Z-octenylesters,process for their preparation and their use
Esters 5-Z-octènyles,procédé pour leur préparation et leur emploi

(30) Priorität: 05.06.1996 DE 19622570
(43) Veröffentlichungstag der Anmeldung: 10.12.1997
(73) Patentinhaber: HAARMANN & REIMER GMBH, D-37601 Holzminden (DE)
(72) Erfinder: Bertram, Heinz-Jürgen, DR., Teterboro, NJ 07608 (US); Güntert, Matthias, Dr., 37603 Holzminden (DE); Kindel, Günter, 37671 Höxter (DE)
(74) Vertreter: Zobel, Manfred, Dr.

(56) Entgegenhaltungen:
- CH-A- 556 144
- US-A- 4 658 071

## Beschreibung

Die Erfindung betrifft neue 5-Z-Octenylester, ein Verfahren zu ihrer Herstellung und ihre Verwendung als Aromastoffe.

Es wurde gefunden, daß die 5-Z-Octenylester ausgewählter Carbonsäuren wertvolle organoleptische Eigenschaften besitzen.

Gegenstand der Erfindung sind Verbindungen der Formel 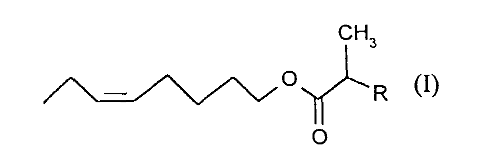 worin
- R: für Wasserstoff, Methyl oder Ethyl steht.

Die Verbindungen I können durch Veresterung von 5-Z-Octenol mit Carbonsäuren, Carbonsäurehalogeniden oder Carbonsäureanhydriden hergestellt werden.

Weiterer Gegenstand der Erfindung ist also ein Verfahren zur Herstellung der Verbindungen I durch Veresterung von 5-Z-Octenol-1 mit einer Verbindung der Formel 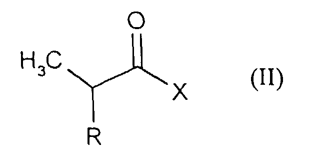 worin
- X: für Wasserstoff, Chlor, Brom oder Iod steht, oder mit einer Verbindung der Formel 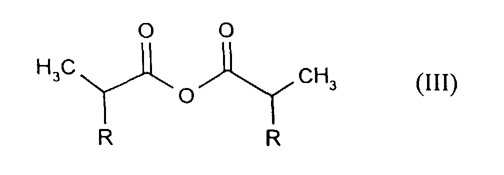
wobei R jeweils die oben angegebene Bedeutung besitzt.

Die allgemeinen Reaktionsbedingungen für Veresterungen von Alkoholen mit Säuren sind bekannt (Organikum, 18. Auflage, Deutscher Verlag der Wissenschaften, Berlin 1990; L.F. Tietze, Th. Eicher: Reaktionen und Synthesen, G. Thieme Verlag, Stuttgart, New York 1991).

Die Veresterung kann bei Temperaturen von 0°C bis 200°C, vorzugsweise von 20°C bis 150°C, gegebenenfalls in inerten Lösungsmitteln wie Ethern, vorzugsweise Methyl-tert.-butylether oder Tetrahydrofuran, in aromatischen und (cyclo)aliphatischen Kohlenwasserstoffen, vorzugsweise Toluol, Xylol, n-Hexan, n-Heptan, Cyclohexan, Methylcyclohexan oder Decalin oder in Estern, vorzugsweise Essigsäureethylester, lester, Essigsäurepropylester, ester, Essigsäurebutyl ester, Propionsäuremethylester, Propionsäureethylester, Propionsäurepropylester oder Propionsäurebutylester bei Normaldruck oder unter Druck, gegebenenfalls in Gegenwart von Basen oder Säuren, durchgeführt werden.

Die erfindungsgemäßen Verbindungen (I) sind wertvolle Aromastoffe; sie zeichnen sich durch ein intensives Mangoaroma aus. Dies ist umso überraschender, als den bisher bekannten 5-Z-Octenylestern das typische, intensive Mangoaroma eindeutig fehlt.

So weisen z.B. 5-Z-Octenylacetat, -3-methylbutyrat und -pentanoat ein typisches Bananenaroma auf (R.G. Berger, F. Drawert, H. Kollmannsberger, Chem. Mikrobiol., Technol. Lebensm. (1986) 10, 120-4; H. Shiota, J. Agric. Food Chem. 41, (1993) 2056; JPN Kokai Tokkyo Koho 7970, 208 (1979).

Die von einem Testpanel aus 6 speziell geschulten Prüfern ermittelten Geschmacksbeschreibungen für die einzelnen erfindungsgemäßen Verbindungen (I) in 0,5 gew.-%iger wäßriger Kochsalzlösung lauten:
5-Z-Octenylpropionat:
   bei einem Zusatz von 7,5 ppm: Melone, Mango, Gurke, fettig
5-Z-Octenylisobutyrat:
   bei einem Zusatz von 7,5 ppm: Mango, grün, Melone
5-Z-Octenyl-2-methylbutyrat:
   bei einem Zusatz von 7,5 ppm: Melone, Fülle, weich, Gurke

Mit ihrem spezifischen Mangoaroma wirken die erfindungsgemäßen Verbindungen (I) in Frucht- und Aromakompositionen geschmacksverstärkend und -abrundend.

Die unter Verwendung der erfindungsgemäßen Verbindungen hergestellten Aromakompositionen können im gesamten Nahrungs- und Genußmittelbereich eingesetzt werden. Insbesondere sind sie für Fettmassen, Backwaren, Fruchtzubereitungen, Saftkonzentrate, Speiseeis und Fruchtkonserven geeignet.

Die erfindungsgemäßen 5-Z-Octenylester werden vorzugsweise in Mengen von 1 ppm bis 1 % Gew.-%, insbesondere 5 ppm bis 500 ppm, bezogen auf das verzehrfertige Nahrungsmittel, verwendet.

Bei den in den Beispielen verwendeten Prozent-Angaben handelt es sich um Gewichtsprozente.

### Beispiele

### Beispiel 1

### Propionsäure-5-Z-octenylester

Man legt bei 0°C in 50 ml Diethylether 5 g 5-Z-Octenol, 5,3 g Dimethylbenzylamin und 0,1 Dimethylaminopyridin vor. Bei dieser Temperatur werden dann 4,0 g Propionylchlorid im Verlauf von 30 Minuten zugetropft. Nach beendeter Zugabe wird noch für 16 Stunden bei Raumtemperatur nachgerührt. Zur Aufarbeitung wird auf verdünnte Schwefelsäure gegeben, einmal mit Ether gewaschen, die vereinigten organischen Phasen zweimal mit Wasser gewaschen, getrocknet und eingeengt. Man erhält 6 g Rohprodukt; nach Kugelrohrdestillation erhält man 5,4 g eines Produktes, das nach GC eine Reinheit von 95 % aufweist.
IR-Spektrum (Film):

| Wellenzahl [cm⁻¹] | |
|---|---|
| 807,6 | w |
| 1029,6 | w |
| 1087,4 | m |
| 1196,1 | s |
| 1352,4 | m |
| 1462,2 | w |
| 1736,1 | s |
| 2871,2 | m |
| 2960,1 | m |
| 3001,7 | m |

(Intensität der IR-Banden: w = schwach, m = mittel, s = stark)

### Beispiele 2 und 3

### Iso- und 2-Methyl-buttersäure-5-Z-octenylester

Bei Verwendung von Isobuttersäurechlorid bzw. 2-Methylbuttersäurechlorid anstelle von Propionsäurechlorid wurden 5-Z-Octenyl-isobutyrat bzw. 5-Z-Octenyl-2-methylbutyrat erhalten.
IR-Spektrum (Film):

| Isobuttersäure-5-Z-octenylester | | 2-Methylbuttersäure-5-Z-octenylester | |
|---|---|---|---|
| Wellenzahl [cm⁻¹] | | | |
| 1162 | s | 1158,7 | m |
| 1199,6 | s | 1190,3 | m |
| 1346,8 | m | 1266,5 | w |
| 1389,2 | w | 1357,6 | w |
| 1467,2 | m | 1461,4 | m |
| 1731,8 | s | 1730,8 | s |
| 2872,1 | m | 2873,3 | m |
| 2938,6 | m | 2935,9 | m |
| 2963,9 | m | 2962,8 | s |
| 3004,7 | m | 3007,1 | m |

### Anwendungsbeispiel

| | Gewichtsteile |
|---|---|
| Dimethylsulfid | 5 |
| Canyophyllen | 10 |
| α-Pinen | 30 |
| Nerol | 30 |
| 3-Z-Hexenylacetat | 35 |
| Acetylmethylcarbinol | 50 |
| 2,5-Dimethyl-4-hydroxy-3(2H)-furanon | 60 |
| 3-Z-Hexenol | 100 |
| Hexanol | 110 |
| γ-Decalacton | 180 |
| Verbindung I | 10-100 |
| Propylenglycol | 290-380 |
| | 1000 |

## Patentansprüche

1. Verbindungen der Formel 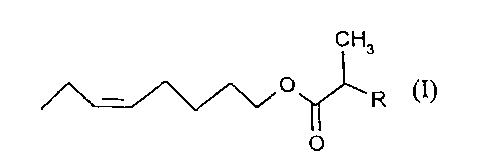 worin
R für Wasserstoff, Methyl oder Ethyl steht.

2. Verfahren zur Herstellung der Verbindungen nach Anspruch 1 durch Veresterung von 5-Z-Octenol-(1) mit einer Verbindung der Formel 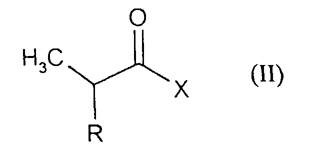 worin
X für Wasserstoff, Chlor, Brom oder Iod steht, oder mit einer Verbindung der Formel 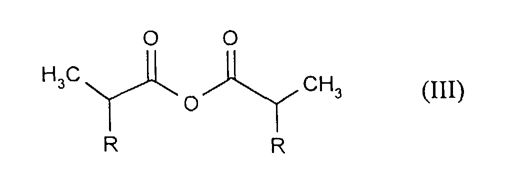
wobei R jeweils die in Anspruch 1 angegebene Bedeutung besitzt.

3. Verwendung der Verbindungen I nach Anspruch 1 als Aromastoffe.

## Claims

1. Compounds of the formula 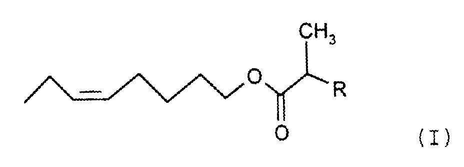 in which
R represents hydrogen, methyl or ethyl.

2. Process for the preparation of the compounds according to Claim 1 by esterification of 5-Z-octen-1-ol with a compound of the formula 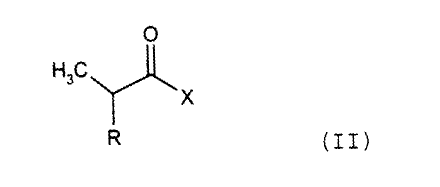 in which
X represents hydrogen, chlorine, bromine or iodine, or with a compound of the formula 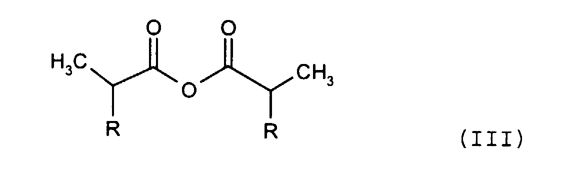 where R in each case has the meaning specified in Claim 1.

3. Use of the compounds I according to Claim 1 as aroma substances.

## Revendications

1. Composés de formule 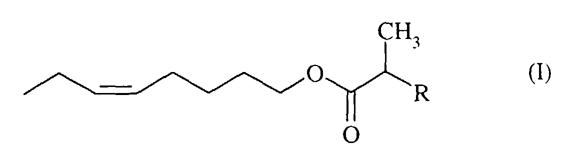 dans laquelle
R représente l'hydrogène, un groupe méthyle ou éthyle.

2. Procédé de préparation des composés selon la revendication 1 par estérification du 5-Z-octénol-1 à l'aide d'un composé de formule 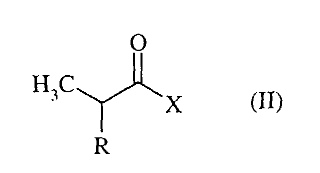 dans laquelle
X représente l'hydrogène, le chlore, le brome ou l'iode, ou à l'aide d'un composé de formule 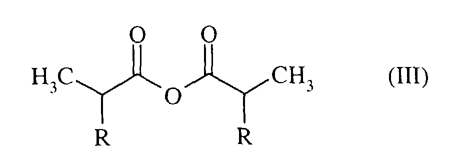 dans laquelle chacun des symboles R a l'une des significations indiquées dans la revendication 1.

3. Utilisation des composés I selon la revendication 1 en tant que matières aromatiques.
